# EUROPÄISCHE PATENTSCHRIFT

(11) **EP 0 609 244 B1**
(45) Veröffentlichungstag und Bekanntmachung des Hinweises auf die Patenterteilung: **23.12.1998**
(21) Anmeldenummer: 92920080.6
(22) Anmeldetag: 23.09.1992
(51) Int. Cl.: C12P 7/42

(54) **VERFAHREN ZUR HERSTELLUNG VON HALOGENIERTEN HYDROXYPHENYLESSIGSÄUREN**
METHOD OF PREPARING HALOGENATED HYDROXYPHENYLACETIC ACIDS
PROCEDE DE FABRICATION D'ACIDES HYDROXYPHENYLACETIQUES HALOGENES

(30) Priorität: 22.10.1991 DE 4134775
(43) Veröffentlichungstag der Anmeldung: 10.08.1994
(73) Patentinhaber: BASF Aktiengesellschaft, 67063 Ludwigshafen (DE)
(72) Erfinder: STAUDENMAIER, Horst, Ralf, D-67134 Birkenheide (DE); HAUER, Bernhard, D-6701 Fussgoenheim (DE); LADNER, Wolfgang, D-6701 Fussgoenheim (DE); MUELLER, Ursula, D-6701 Fussgoenheim (DE); PRESSLER, Uwe, D-6701 Altrip (DE); MEYER, Joachim, D-6701 Maxdorf (DE)
(86) Internationale Anmeldenummer: EP9202194
(87) Internationale Veröffentlichungsnummer: WO9308294

(56) Entgegenhaltungen:
- EP-A- 0 089 039
- Agric. Biol. Chem., Band. 49, Nr. 3, 1985 Fumikie Yoshizako et al.: "The Formation of 2,6-Dihydroxyphenylacetic Acid from Phenylacetic Acid by Various Fungi ",
- NIPPON NOGEIKAGAKU KAISHI, vol.56, no.12, 1982, page 1135 - 1141, F. YOSHIZAKO ET AL. ''

## Beschreibung

Die Erfindung betrifft ein neues Verfahren zur fermentativen Herstellung von Verbindungen der allgemeinen Formeln Ia und Ib 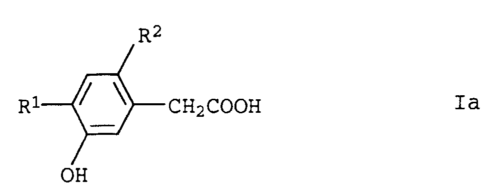 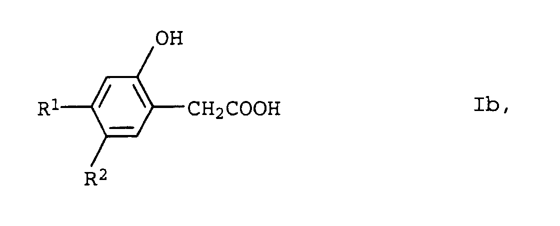 worin
- R¹: ein Wasserstoff-, ein Fluor-, ein Chlor- oder ein Bromatom und
- R²: ein Fluor- oder ein Chloratom bedeuten.

Verbindungen der allgemeinen Formeln Ia und Ib sind wertvolle Zwischenprodukte bei der Farbstoff- und Pharmazeutikaherstellung.

Es ist bekannt, daß Mikroorganismen aromatische Verbindungen hydroxylieren können. Die Hydroxylierung des aromatischen Rings stellt in vielen Fällen den ersten Schritt einer Reaktionsfolge dar, die zum Abbau der betreffenden Substanz führt.

Von Yoshizako et al. (Agric. Biol. Chem. 49 (3), 1985, 877 bis 879) ist bekannt, daß der Abbau von Phenylessigsäure bei verschiedenen Pilzen über das Zwischenprodukt 2,5-Dihydroxyphenylessigsäure (Homogentisinsäure) verläuft. Hierzu zählen beispielsweise Pilze der Gattungen Aspergillus, Fusarium, Gibberella, Mucor, Pellicularia, Penicillium, Phellinus und Rhizopus. Homogentisinsäure als Stoffwechselprodukt von Phenylessigsäure fällt jedoch nur in sehr geringen Mengen an, da sie in der Regel durch Spaltung des aromatischen Rings weiter abgebaut wird.

In EP 089 039 wird ein mikrobielles Verfahren zur Herstellung von D-β-Hydroxyalktansäuren aus Alkansäuren oder 2-Alkensäuren beschrieben.

Es bestand daher die Aufgabe, ein fermentatives Herstellungsverfahren für Verbindungen der Formeln Ia und Ib bereitzustellen, das von leicht zugänglichen Ausgangsverbindungen ausgeht und gute Ausbeuten liefert.

Demgemäß wurde gefunden, daß das eingangs definierte Verfahren zur fermentativen Herstellung von Verbindungen der allgemeinen Formeln Ia und Ib 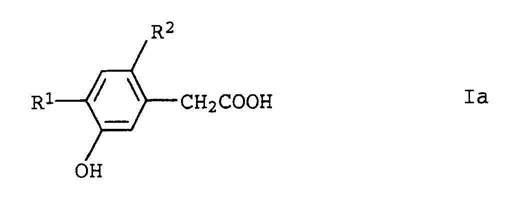 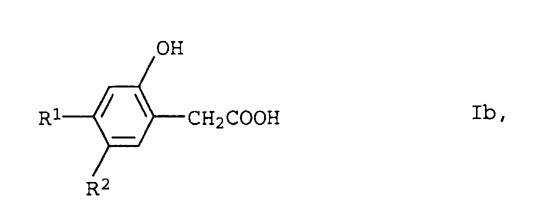 worin
- R¹: ein Wasserstoff-, ein Fluor-, ein Chlor- oder ein Bromatom und
- R²: ein Fluor- oder ein Chloratom bedeuten,
besonders gute Ausbeuten liefert, wenn man Verbindungen der allgemeinen Formel II 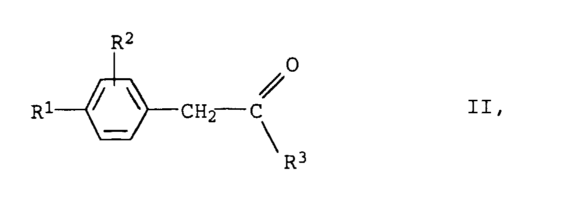 worin
- R¹, R²: die obengenannten Reste und
- R³: eine Hydroxylgruppe oder eine Methoxygruppe oder eine Aminogruppe bedeuten,
in Gegenwart eines Mikroorganismus hydroxyliert, der Verbindungen der Formel II hydroxylieren, jedoch nicht als C-Quelle verwerten kann.

Die für das erfindungsgemäße Verfahren benötigten Ausgangsverbindungen der allgemeinen Formel II sind bekannt.

Sie sind beispielsweise nach in Houben-Weyl, "Methoden der organischen Chemie", Band 8 beschriebenen Verfahren herstellbar.

Die Umwandlung der oben genannten Ausgangsverbindungen II zu Verbindungen der allgemeinen Formeln Ia und Ib durch Mikroorganismen beinhaltet eine Hydroxylierung in para-Stellung zu Rest R². Befindet sich der Rest R² in der 2-Position der Ausgangsverbindung so erhält man durch das erfindungsgemäße Verfahren ein 2-Halogen-5-hydroxyderivat mit der allgemeinen Formel Ia.

Befindet sich der Rest R² in der 3-Position der Ausgangsverbindung so erhält man ein 5-Halogen-2-hydroxyderivat mit der allgemeinen Formel Ib.

Reste an der 4-Position des aromatischen Kerns wie ein Wasserstoff- oder ein Halogenatom werden durch das erfindungsgemäße Verfahren nicht verändert. Bevorzugt werden Ausgangsverbindungen verwendet, die an der 4-Position ein Wasserstoffatom tragen.

Neben der Hydroxylierung am Aromaten kann auch eine Veränderung an der Carboxylgruppe durch das erfindungsgemäße Verfahren stattfinden. So wird ein Methylester oder ein Säureamid zur freien Säure bzw. deren Salz umgewandelt. Wird als Ausgangsverbindung eine freie Säure bzw. deren Salz verwendet, so wird diese Gruppe nicht durch das Verfahren verändert.

Die für das erfindungsgemäße Verfahren geeigneten Mikroorganismen müssen einerseits die Fähigkeit besitzen, die Ausgangsverbindungen am aromatischen Kern hydroxylieren zu können, andererseits dürfen sie die Ausgangsverbindung nicht als Kohlenstoffquelle benutzen.

Solche Mikroorganismen erhält man zweckmäßigerweise durch Mutationen von Wildtypstämmen, die die Fähigkeit zur Aromatenhydroxylierung besitzen.

Bevorzugt werden als Wildtypstämme Pilze, insbesondere Deuteromyceten verwendet. Besonders bevorzugt sind solche der Gattung Beauveria, insbesondere der Art Beauveria bassiana (DSM 6650).

Ob ein Mikroorganismus geeignet ist, die Ausgangsverbindung in gewünschter Weise am aromatischen Kern zu hydroxylieren, läßt sich leicht mit Hilfe analytischer Methoden, z.B. der Gaschromatographie, anhand des Nährmediums bestimmen.

Zweckmäßigerweise setzt man dem Nährmedium die zu hydroxylierende Ausgangsverbindung zu und überprüft im Laufe der Züchtung, ob die Ausgangsverbindung abnimmt und die gewünschte hydroxylierte Verbindung als Metabolit auftritt. Zur Identifizierung von Metaboliten können übliche Verfahren wie "resting-cell"-Experimente, Einsatz von Hemmstoffen oder Isotopenmarkierung verwendet werden.

Für das erfindungsgemäße Verfahren geeignete Mikroorganismen lassen sich auch auffinden, in dem man die Fähigkeit zur Aromatenhydroxylierung nicht an der gewünschten Ausgangsverbindung selbst, sondern an einem mit der gewünschten Ausgangsverbindung strukturell verwandten Molekül überprüft. Beispielsweise kann die Umsetzung von Phenylessigsäure zu 2,5-Dihydroxyphenylessigsäure (Homogentisinsäure) als ein Kriterium für zur Hydroxylierung geeignete Mikroorganismen verwendet werden.

Von den geeigneten Mikroorganismen werden zweckmäßigerweise Mutanten erzeugt, die nicht mehr in der Lage sind, die Ausgangsverbindung als Kohlenstoffquelle zu verwenden.

Zur Erzeugung solcher Mutanten können bekannte mikrobiologische Techniken eingesetzt werden. Zur Auslösung von Mutationen können alle gängigen Methoden verwendet werden wie die Anwendung von mutagenen Substanzen, z.B. Nitrosoguanidin, Ethylmethansulfonat, Natriumnitrit, oder die Einwirkung von elektromagnetischer Strahlung wie UV-, Gamma- oder Röntgenstrahlung. Weiterhin können zur Mutagenese auch transponierbare genetische Elemente verwendet werden. Zur Isolierung der Mutanten können verschiedene Eigenschaften herangezogen werden, wie die Unfähigkeit, auf Phenylessigsäure als einziger C-Quelle zu wachsen oder die optisch erkennbare Braunfärbung infolge der gebildeten Homogentisinsäure. Gegebenenfalls kann an dieser Stelle auch noch eine Anreicherung der gesuchten Mutanten durchgeführt werden.

Das erfindungsgemäße Verfahren wird mit geeigneten Mikroorganismen durchgeführt, die in einem Nährmedium, das die Ausgangsverbindung in einer Konzentration von 1 bis 20 g/l, bevorzugt von 5 bis 15 g/l enthält, gezüchtet werden.

Die Züchtungsdauer hängt von der Ausgangsverbindung und dem Mikroorganismus ab; sie beträgt in der Regel einige Tage. Zweckmäßigerweise wird die Züchtung so lange fortgesetzt, bis ein nahezu quantitativer Umsatz der Ausgangsverbindung erfolgt ist.

Die Züchtung kann in einem kontinuierlichen oder einem diskontinuierlichen Verfahren betrieben werden; bevorzugt wird jedoch eine diskontinuierliche Verfahrensweise.

Die Isolierung und Reinigung der halogenierten Hydroxyphenylessigsäure aus dem Nährmedium kann nach bekannten Methoden erfolgen. Zweckmäßigerweise trennt man die feste Biomasse vom Nährmedium ab, extrahiert den Wertstoff z.B. mit einem organischen Lösungsmittel und isoliert den Wertstoff aus der extrahierten Phase, gegebenenfalls nach weiterer Reinigung, z.B. durch Kristallisation.

Die Erfindung wird durch die folgenden Beispiele weiter veranschaulicht.

### Beispiel 1

### Herstellung einer Mutante (LU 6577) aus Beauveria bassiana

Der Pilz Beauveria bassiana DSM 6650 wurde mit Hilfe von N-Methyl-N'-nitro-N-nitrosoguanidin (MNNG) mutagenisiert. Eine Sporensuspension des Pilzes in 0,1 M Phosphatpuffer pH 7,0, 0,1 Gew.-% Polyethoxysorbitanoleat (Tween 80®) wurde auf einen Titer von 10⁷ Sporen pro ml eingestellt. 10 ml dieser Sporensuspension wurden durch Zugabe einer Stammlösung von 5 mg/ml MNNG (gelöst in Dimethylformamid) auf eine Konzentration von 0,2 mg/ml MNNG eingestellt und 15 min unter leichtem Schütteln bei 30°C inkubiert. Dann wurden die Sporen durch Zentrifugation (5 Minuten mit 5000 Upm) geerntet und zweimal mit 10 ml Tweenpuffer gewaschen. Die Sporen wurden in Tweenpuffer aufgenommen, verdünnt und auf Komplexmedium ausplattiert. Der Vergleich mit unbehandelten Sporen ergab bei mehreren Experimenten regelmäßig eine Überlebensrate der mutagenisierten Sporen von 1 bis 2 %.

Die mutagenisierten Sporen wurden auf Agarplatten mit Komplexmedium der folgenden Zusammensetzung ausplattiert und 5 Tage bei 30°C inkubiert:

| | |
|---|---|
| 50 g/l | D-Glucose |
| 10 g/l | Hefeextrakt |
| 3,6 g/l | K₂HPO₄ |
| 1,5 g/l | KH₂PO₄ |
| 0,5 g/l | MgSO₄ x 7 H₂O |
| 0,05 g/l | MnSO₄ x H₂O |
| 2 ml/l | Spurenelementlösung |
| 20 g/l | Agar |

Spurenelementlösung:

| | |
|---|---|
| 200 mg/l | Eisen(II)sulfat-1-hydrat |
| 10 mg/l | Zink(II)sulfat-4-hydrat |
| 3 mg/l | Manganchlorid-4-hydrat |
| 30 mg/l | Borsäure |
| 20 mg/l | Cobalt(II)chlorid-6-hydrat |
| 1 mg/l | Kupfer(II)chlorid-2-hydrat |
| 2 mg/l | Nickel(II)chlorid-6-hydrat |
| 3 mg/l | Natriummolybdat-2-hydrat |
| 500 mg/l | Ethylendiamintetraessigsäure (EDTA) |

Von den entstandenen Einzelkolonien wurde mit Hilfe von Zahnstochern ein kleines Mycelstück in Reagenzgläser mit je 2 ml des folgenden Minimalmediums mit Phenylessigsäure als einziger Kohlenstoffquelle angeimpft:

| | |
|---|---|
| 5 g/l | Phenylessigsäure |
| 5 g/l | (NH₄)₂SO₄ |
| 3,6 g/l | K₂HPO₄ |
| 1,5 g/l | KH₂PO₄ |
| 0,5 g/l | MgSO₄ x 7 H₂O |
| 0,05 | g/l MnSO₄ x H₂O |
| 2 ml/l | Spurenelementlösung |

Die Ansätze wurden 7 Tage bei 30°C geschüttelt (180 Upm). Klone, die während dieses Zeitraums das Medium nicht dicht bewachsen hatten, wurden weiter charakterisiert. Die Anteile der nicht gewachsenen Klone betrug bei verschiedenen Experimenten zwischen 3 und 8 %.

Die weiter zu charakterisierenden Klone wurden aus dem Rückstellmuster in je 2 ml Komplexmedium mit 2 g/l Phenylessigsäure angeimpft und 10 Tage bei 30°C schüttelnd gezüchtet.

Dann wurden die Kulturen abzentrifugiert und mit dem Kulturüberstand ein photometrischer Test durchgeführt:
Zu 0,5 ml Kulturüberstand wurden 0,5 ml 1,6 % NaNO₂ und 0,2 ml 1 M H₂SO₄ gegeben und 10 min inkubiert. Dann wurden 0,25 ml 0,2 Gew.-% EDTA in 2,3 M NaOH zugegeben und die Proben bei 450 nm gegen den Leerwert gemessen. Für den Leerwert wurde bei sonst gleichem Vorgehen anstatt NaNO₂-Lösung Wasser zugegeben.

Klone, die im photometrischen Test eine Extinktion A450 > 1,8 hatten (1-5 % der getesteten Klone), wurden in Schüttelkolbenexperimenten weiter untersucht. Dazu wurde jeweils eine Vorkultur der Klone in Komplexmedium mit 2,5 g/l Phenylessigsäure angesetzt (30 ml in 250 ml-Erlenmeyerkolben). Nach 3 Tagen Schütteln bei 30°C wurde mit je 5 ml der Vorkultur eine Hauptkultur mit 10 g/l Phenylessigsäure angeimpft und bei 30°C geschüttelt. Nach jeweils 3 und 7 Tagen wurde eine Probe gezogen und der Gehalt an Phenylessigsäure und deren Derivaten gaschromatographisch analysiert.

Es wurde 1 ml der Kulturen abgenommen, mit 100 µl 5 M HCl und 800 µl Essigester versetzt, 15 s gemischt und 2 min bei 12000 g zentrifugiert. 50 µl der organischen Phase wurden abgenommen und mit 50 µl N-Methyl-N-(trimethylsilyl)-trifluoracetamid (MSTFA) versetzt.

Die Proben wurden gaschromatographisch aufgetrennt (165°C isotherm, Säule: Methylsilicon 12,5 m, Hewlett-Packard, 1 µl Injektionsvolumen). Phenylessigsäure erschien im Chromatogramm nach 1,7 min, Homogentisinsäure nach 7,8 min.

Die Mutante Lu 6577 zeigte in diesem Test nach 7 Tagen vollständigen Umsatz von Phenylessigsäure zu Homogentisinsäure.

### Beispiel 2

### Herstellung von 2-Chlor-5-hydroxyphenylessigsäure aus 2-Chlorphenylessigsäure

Die Mutante Lu 6577 wurde in 5 x 330ml des folgenden Mediums in 1 l-Erlenmeyerkolben angeimpft und 3 Tage bei 30°C mit 180 Upm aerob schüttelnd inkubiert:

| | |
|---|---|
| 0,5 g/l | 2-Chlorphenylessigsäure |
| 50 g/l | D-Glucose |
| 10 g/l | Hefeextrakt |
| 0,5 g/l | MgSO₄ x 7 H₂O |
| 1,5 g/l | KH₂PO₄ |
| 3,6 g/l | K₂HPO₄ |
| 2 ml/l | Spurenelementlösung |

Mit dieser Vorkultur wurde ein 10-1-Fermenter mit demselben Medium und einer 2-Chlorphenylessigsäurekonzentration von 5 g/l angeimpft. Der Fermenter wurde mit 600 Upm gerührt und mit 1 Volumen Luft pro Volumen Reaktor pro Minute begast. Als die Umsetzung abgeschlossen war, wurden die Zellen abzentrifugiert. Der Kulturüberstand wurde mit HCl auf pH = 2 gestellt und die gebildete 2-Chlor-5-hydroxyphenylessigsäure durch Extraktion mit Essigsäureethylester gewonnen. Ausbeute und Fermentationsdauer sind in Tabelle 1 aufgeführt.

### Beispiel 3

### Herstellung von 5-Chlor-2-hydroxyphenylessigsäure aus 3-Chlorphenylessigsäure

Die Umwandlung wurde wie in Beispiel 2 beschrieben durchgeführt. Als Ausgangsverbindung wurde 3-Chlorphenylessigsäure eingesetzt. Das Ergebnis ist in Tabelle 1 beschrieben.

### Beispiel 4

### Herstellung von 2-Fluor-5-hydroxyphenylessigsäure aus 2-Fluorphenylessigsäure

Die Umwandlung wurde wie in Beispiel 2 beschrieben durchgeführt. Als Ausgangsverbindung wurde 2-Fluorphenylessigsäure in einer Konzentration von 0,5 g/l (Vorkultur) und 3 g/l (Hauptkultur) eingesetzt. Das Ergebnis ist in Tabelle 1 aufgeführt.

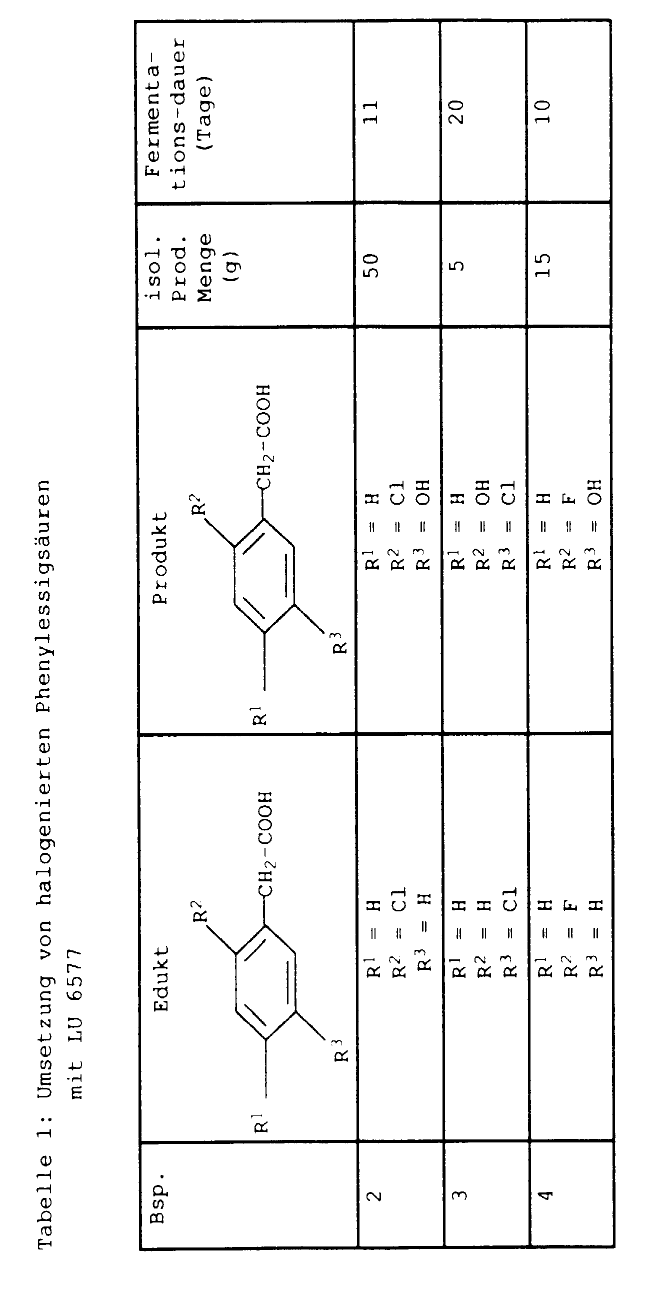

## Patentansprüche

1. Verfahren zur fermentativen Herstellung von Verbindungen der allgemeinen Formeln Ia und Ib 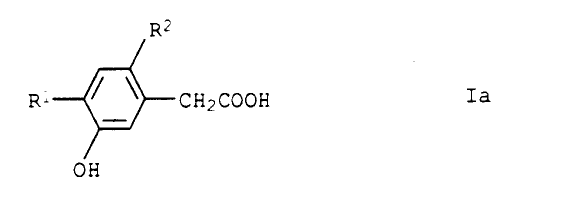 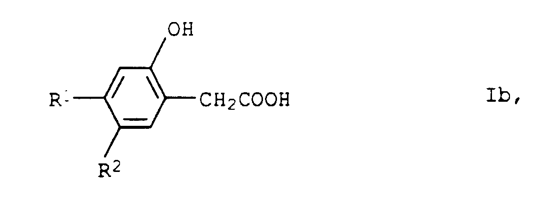 worin
R¹ ein Wasserstoff-, ein Fluor-, ein Chlor- oder ein Bromatom und
R² ein Fluor- oder ein Chloratom bedeuten,
dadurch gekennzeichnet, daß man Verbindungen der allgemeinen Formel II 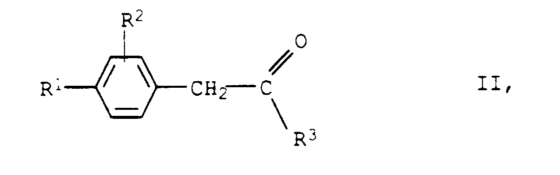 worin
R¹, R² die obengenannten Reste und
R³ eine Hydroxylgruppe oder eine Methoxygruppe oder eine Aminogruppe bedeuten,
in Gegenwart eines Mikroorganismus hydroxyliert, der Verbindungen der Formel II hydroxylieren, jedoch nicht als C-Quelle verwerten kann.

2. Verfahren nach Anspruch 1, dadurch gekennzeichnet, daß als Mikroorganismus ein Pilz verwendet wird.

3. Verfahren nach Anspruch 2, dadurch gekennzeichnet, daß als Pilz ein Vertreter der Deuteromyceten verwendet wird.

4. Verfahren nach Anspruch 2, dadurch gekennzeichnet, daß als Mikroorganismus ein Pilz der Gattung Beauveria verwendet wird.

## Claims

1. A process for the preparation, by fermentation, of compounds of the formulae Ia and Ib 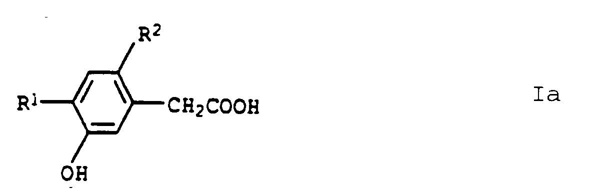 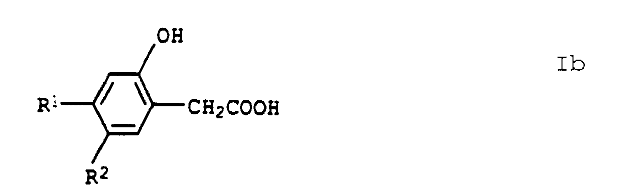 where
R¹ is hydrogen, fluorine, chlorine or bromine, and
R² is fluorine or chlorine,
which comprises hydroxylating compounds of the formula II 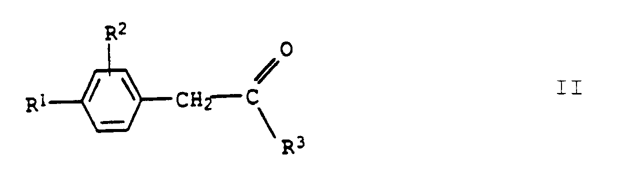 where
R¹ and R² have the abovementioned meanings, and
R³ is hydroxyl, methoxy or amino,
in the presence of a microorganism which is able to hydroxylate, but not utilize as C source, compounds of the formula II.

2. A process as claimed in claim 1, wherein a fungus is used as microorganism.

3. A process as claimed in claim 2, wherein a representative of the Deuteromycetes is used as fungus.

4. A process as claimed in claim 2, wherein a fungus of the genus Beauveria is used as microorganism.

## Revendications

1. Procédé pour préparer par fermentation des composés de formules générales Ia et Ib 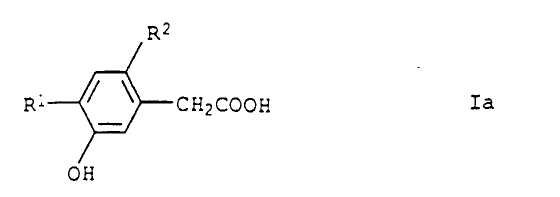 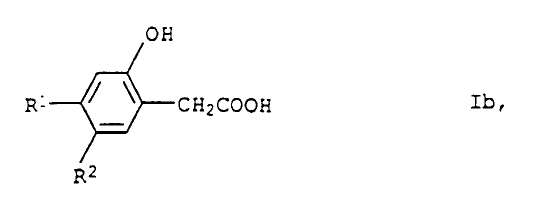 dans lesquelles
R¹ représente un atome d'hydrogène, de fluor, de chlore ou de brome et
R² représente un atome de fluor ou de chlore,
caractérisé par le fait que l'on hydrolyse des composés de formule générale II 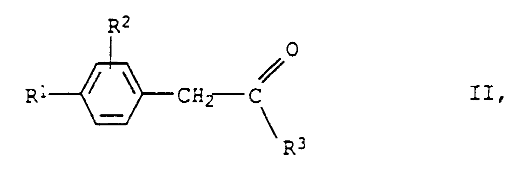 dans laquelle
R¹ et R² ont les significations indiquées ci-dessus et
R³ représente un groupe hydroxy ou méthoxy ou amino,
en présence d'un microorganisme capable d'hydroxyler les composés de formule II mais non de les valoriser en tant que source de carbone.

2. Procédé selon la revendication 1, caractérisé par le fait que le microorganisme utilisé est un mycète.

3. Procédé selon la revendication 2, caractérisé par le fait que le mycète appartient à la famille des deutéromycètes.

4. Procédé selon la revendication 2, caractérisé par le fait que le microorganisme appartient au genre Beauvaria.
